(19) Europäisches Patentamt
European Patent Office
Office européen des brevets

(11) Veröffentlichungsnummer: **0 394 850 A1**

(12) # EUROPÄISCHE PATENTANMELDUNG

(21) Anmeldenummer: **90107481.5**

(51) Int. Cl.5: **C07D 265/08**

(22) Anmeldetag: **19.04.90**

(30) Priorität: **28.04.89 DE 3914155**

(43) Veröffentlichungstag der Anmeldung: **31.10.90 Patentblatt 90/44**

(84) Benannte Vertragsstaaten: **GR**

(71) Anmelder: **Henkel Kommanditgesellschaft auf Aktien**
**Henkelstrasse 67**
**D-4000 Düsseldorf 13(DE)**

(72) Erfinder: **Leinen, Hans Theo, Dr.**
**Gertrudisstrasse 2**
**D-4000 Düsseldorf(DE)**
Erfinder: **Fristad, William E., Dr.**
**321 Buena Vista Dr.**
**Santa Rosa CA 95404(US)**
Erfinder: **Neumann, Peter**
**Nosthoffenstrasse 61**
**D-4000 Düsseldorf(DE)**

(54) **Verfahren zur Herstellung von 5,6-Dihydro-4H-1,3-oxazinen.**

(57) Ein Verfahren zur Herstellung von 2-substituierten 5,6-Dihydro-4H-1,3-oxazinen liefert diese Verbindungen in hohen Ausbeuten, wenn man Carbonsäuren, Carbonsäureester oder Carbonsäureglyceride mit 3-Amino-1-propanol oder 3-Hydropropyl-amide dieser Carbonsäuren in Gegenwart von Titan-bzw. Zirkoniumverbindungen des Typs M $(OR^3)_4$ (M = Ti bzw. Zr) umsetzt.

EP 0 394 850 A1

Xerox Copy Centre

## Verfahren zur Herstellung von 5,6-Dihydro-4H-1,3-oxazinen

Die Erfindung betrifft ein Verfahren zur Herstellung von 5,6-Dihydro-4H-1,3-oxazinen der allgemeinen Formel I

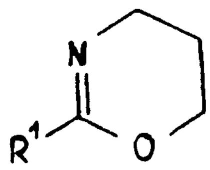

(I)

in der $R^1$
ein gegebenenfalls hydroxy, vicinal dihydroxy- oder vicinal hydroxy, $C_1$-$C_2$-alkoxysubstituierter Kohlenwasserstoffrest mit mindestens 4, insbesondere 7 bis 21 Kohlenstoffatomen in der Kohlenwasserstoffkette,
ein gegebenenfalls im aromatischen Kern substituierter Aryl-oder Aralkylrest,
eine hydroxysubstituierte Alkylgruppe mit 1 bis 3 Kohlenstoffatomen,
eine alkoxysubstituierte Alkylgruppe mit 1 bis 6 Kohlenstoffatomen in der Alkyl- und 1 bis 18 Kohlenstoffatomen in der Alkoxygruppe oder
einen Rest der allgemeinen Formel II
$R^2$-$(OC_2H_5)_p$-O-$CH_2$- (II)
in der
$R^2$ eine Alkyl- oder Alkenylgruppe mit 1 bis 18 Kohlenstoffatomen, insbesondere eine Methylgruppe, und
p eine Zahl im Bereich von 1 bis 10, insbesondere 1 bis 2, ist,
bedeutet,
durch Kondensation von Carbonsäure-(3-hydroxypropyl)-amiden bzw. deren Vorläufern in Gegenwart von Katalysatoren in flüssiger Phase, wobei unter Vorläufern als erste Komponenten Carbonsäuren und Ester von Carbonsäuren mit niederen Alkanolen oder Glycerin und als zweite Komponente 3-Amino-1-propanol zu verstehen sind.

In 2-Stellung substituierte 5,6-Dihydro-4H-1,3-oxazine sind bekannte Verbindungen, die unter anderem in Form ihrer Polymere Kunststoffen als antistatische Mittel zugesetzt werden können, vgl. US-C 3 470 267. Ihr Einsatz zusammen mit Zinkstabilisatoren in PVC-Massen ist in der US-C 4 755 549 und in der DE-A 36 30 318 beschrieben.

Die vorgenannten 5,6-Dihydro-4H-1,3-oxazine, die im folgenden kurz als Dihydrooxazine bezeichnet werden, können auf verschiedenen Wegen hergestellt werden, z.B. gemäß Ullmanns Encyclopädie der technischen Chemie, 4. Auflage, Band 19, Seite 437 (1980), Verlag Chemie Weinheim, durch Umsetzung von Carbonsäurenitrilen mit 3-Amino-1-propanol oder gemäß US-C 3 483 141 durch Umsetzen von Carbonsäure-(3-hydroxypropyl)-amiden in Gegenwart von Schwefelsäure. Diese Verfahren liefern jedoch insbesondere bei der Herstellung von Dihydrooxazinen mit längerkettigen Substituenten in 2-Stellung keine guten Ausbeuten.

Es wurde nun gefunden, daß sich spezielle Titan- und Zirkoniumverbindungen ausgezeichnet als Katalysatoren in einem Verfahren der eingangs genannten Art eignen, wobei Ausbeuten bis ca. 80 % der Theorie, bezogen auf die Ausgangsverbindung, erzielt werden können.

Demgemäß ist die Erfindung auf ein Verfahren der eingangs genannten Art gerichtet, bei dem man

a) Carbonsäuren der allgemeinen Formel III
$R^1$-COOH (III)
in der $R^1$ wie oben definiert ist, Ester dieser Carbonsäuren mit Monoalkanolen mit 1 bis 4 Kohlenstoffatomen oder Glyceride dieser Carbonsäuren mit 3-Amino-1-propanol oder

b) 3-Hydroxypropyl-amide dieser Carbonsäuren in Gegenwart von Titan- bzw. Zirkoniumverbindungen der allgemeinen Formel IV
$M(OR^3)_4$ (IV)
in der M vierwertiges Titan oder Zirkonium und $R^3$ eine Alkylgruppe mit mindestens 2, insbesondere 2 bis 4 Kohlenstoffatomen, eine Acylgruppe mit mindestens 2, insbesondere 2 bis 10 Kohlenstoffatomen, eine 3-Aminopropylen-1-oxygruppe oder einen Rest eines beta-Diketons der allgemeinen Formel V
$R^4$- C =CH-CO-$R^5$ (V)
in der $R^4$ und $R^5$ gleiche oder verschiedene Reste aus der von Alkylgruppen mit 1 bis 4 Kohlenstoffatomen und gegebenenfalls in p-Stellung substituiertem Phenyl gebildeten Gruppe sind, wobei jeweils zwei der

Gruppen $R^2$ zusammen aus dem zweibindigen Rest eines zweiwertigen Alkohols mit 2 bis 4 Kohlenstoffatomen bestehen können,
in Gegenwart von Titanylacetylacetonat oder
in Gegenwart von Kondensationsprodukten von Titan(IV) bzw. Zirkonium(IV)-tetraalkoxylaten der allgemeinen Formel III, in der M und $R^3$ wie oben definiert sind, mit mehrfunktionellen Alkanolen, insbesondere mit 3 bis 12 Kohlenstoffatomen und 2 bis 6 Hydroxylgruppen,
kondensiert und die so erhaltenen 5,6-Dihydro-4H-1,3-oxazine unter Entfernung des Reaktionswassers bzw. -alkohols isoliert.

Das Verfahren der Erfindung ist unter anderem auf Fettsäure-(3-hydroxypropyl)-amide anwendbar, die von geradkettigen oder verzweigten, gesättigten oder ungesättigten Fettsäuren mit mindestens 4 Kohlenstoffatomen, insbesondere mit 8 bis 22 Kohlenstoffatomen, einschließlich hydroxy- und vicinal dihydroxy- oder vicinal hydroxy, $C_1$-$C_2$-alkoxy-substituierten Derivaten derselben, und auf deren Vorläufer (Fettsäuren, Fettsäureester und Fettsäureglyceride, insbesondere Triglyceride). Besonders bevorzugte Ausgangsverbindungen für das Verfahren der Erfindung sind Fettsäuren natürlichen, z.B. pflanzlichen, tierischen oder synthetischen Ursprungs (einschließlich technischer Gemische derselben) oder deren Derivate.

Typische Vertreter der vorstehend genannten natürlichen Fettsäuren sind Capryl-, Caprin-, Myristin-, Palmitin-, Stearin-, 12-Hydroxystearin-, Arachin-, Behen-, Lignocerin-, Laurolein-, Myristolein-, Palmitolein-, Öl-, Gadolein-, Eruca-, Ricinol-, Linol-, Linolen- und Arachidonsäure. Wie in der Fettchemie üblich, werden meist nicht die reinen Fettsäuren bzw. (3-Hydroxypropyl)-amine, sondern technische Gemische derselben eingesetzt, wie sie aus natürlichen Rohstoffen zugänglich sind, z.B. aus gegebenenfalls gehärtetem Kokosöl, Palmöl, Palmkernöl, Sojaöl, Erdnußöl, Rüböl, Rapsöl, Olivenöl, Leinöl, Sonnenblumenöl, Ricinusöl, Rindertalg, Schweineschmalz und Fischöl.

Im Verfahren der Erfindung können im übrigen auch die vorgenannten natürlichen Fette und Öle, in denen die Fettsäuren als Triglyceride vorliegen, eingesetzt werden.

Vicinal dihydroxy- oder vicinal hydroxy, $C_1$-$C_2$-alkoxy-substituierte Vertreter dieser Fettsäuren lassen sich aus ungesättigten Fettsäuren der vorstehend genannten Art durch Epoxidation und Ringöffnung der Epoxygruppen mit Wasser bzw. Methanol oder Ethanol herstellen, z.B. aus Sojaöl.

Als typische Vertreter in dem Verfahren der Erfindung einsetzbarer synthetischer Fettsäuren sind die durch Oxidation von Olefinen, Paraffinen, Oxoalkoholen und Ziegler-Alkoholen erhältlichen zu nennen.

Weitere in dem Verfahren der Erfindung einsetzbare Carbonsäuren, Carbonsäureester, Carbonsäureglyceride und Carbonsäurepropanolamide können insbesondere die folgenden bzw. von diesen abgeleitet sein:
Benzoesäure, die 1 bis 3 Substituenten aufweisen kann; typische Substituenten sind $C_1$-$C_4$-Alkylgruppen, insbesondere Methyl, $C_1$-$C_4$-Alkoxygruppen, insbesondere Methoxy, sowie Halogenatome wie Chlor und Brom;
Phenylessigsäure, die im aromatischen Kern mit 1 bis 3 der vorgenannten Substituenten substituiert sein kann;
Capron- und Önanthsäure;
Hydroxycarbonsäuren mit 2 bis 7 Kohlenstoffatomen, insbesondere Hydroxyessigsäure (Glykolsäure), Hydroxypropionsäure, Hydroxybuttersäure, Hydroxyvaleriansäure, Hydroxycapronsäure, insbesondere die omega-hydroxysubstituierten Isomere derselben, und gegebenenfalls deren Lactone;
alkoxysubstituierte Carbonsäuren, die formal Alkylierungsprodukte der vorgenannten Hydroxycarbonsäuren sind und 1 bis 18 Kohlenstoffatome in der Alkoxygruppe aufweisen; bevorzugt sind methoxysubstituierte Carbonsäuren;
Ethercarbonsäuren der Formel
$R^2$-$(OC_2H_5)_p$-O-$CH_2$-COOH
in der $R^2$ und p wie oben definiert sind; typische Beispiele hierfür sind Ethercarbonsäuren, die durch katalytische Oxidation von Anlagerungsprodukten von Ethylenoxid an primäre, gesättigte oder gegebenenfalls auch ungesättigte Alkohole mit 1 bis 18 Kohlenstoffatomen wie Methanol, Ethanol, Propanol, Butanol, Pentanol und Hexanol sowie gesättigte oder ungesättigte Fettalkohole einschließlich technischer Gemische derselben wie Capron-, Önanth-, Capryl-, Caprin-, Undecenyl-, Lauryl-, Myristyl-, Cetyl-, Stearyl-, Oleyl-, Elaidyl-, Linoleyl- und Linoleylaklohol, erhalten werden und z.B. in DE-A 26 36 123, EP-B 0 039 111, EP-B 0 018 681 und DE-A 31 35 946 beschrieben sind.

Besonders bevorzugte Ethercarbonsäuren sind Methoxy-ethoxy-und Methoxy-ethoxyethoxyessigsäure.

Die in dem Verfahren der Erfindung einsetzbaren Carbonsäurepropanolamide können nach üblichen Verfahren erhalten werden, z.B. über die Alkylester oder die Säurechloride.

Die als Katalysatoren im Verfahren der Erfindung einsetzbaren Titan- bzw. Zirkoniumverbindungen sind bekannt und größtenteils im Handel erhältlich. Kondensationsprodukte von Titan(IV) bzw. Zirkonium(IV)-tetraalkoxylaten mit mehrfunktionellen Alkanolen mit 3 bis 12 Kohlenstoffatomen und 2 bis 6 Hydroxylgruppen wie Glycerin, Trimethylolpropan und Pentaerythrit sind Veresterungs- und/oder Umesterungskatalysatoren, die z.B. in der US-C 4,705,764, auf deren Inhalt hier bezug genommen wird, beschrieben sind. Ein geeignetes Polyalkanol ist weiterhin Polyvinylalkohol. Die ebenfalls als Katalysatoren geeigneten Titan- bzw. Zirkoniumtetra-(3-aminoprop-1-oxylate) sind aus Titan- bzw. Zirkoniumtetraalkoxylaten und 3-Amino-1-propanol herstellbar.

Gemäß einer bevorzugten Ausführungsform der Erfindung verwendet man als Katalysatoren Ester der Titansäure ($H_4TiO_4$) oder der Zirkonsäure ($H_4ZrO_4$) bzw. gemischte Anhydride der Titan-oder Zirkonsäure mit organischen Säuren der allgemeinen Formel III, in der M = Ti oder Zr und $R^3$ eine Alkylgruppe mit mindestens 2 oder mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten.

Gemäß einer weiteren vorteilhaften Ausführungsform der Erfindung verwendet man Katalysatoren aus der von Titan- bzw. Zirkonium-tetraethylat, -tetrapropylat, -tetraisopropylat, -tetrabutylat und -tetraacetat gebildeten Gruppe.

Gemäß einer weiteren vorteilhaften Ausführungsform der Erfindung verwendet man als Katalysatoren Titan- bzw. Zirkoniumacetylacetonate der allgemeinen Formel (VI)

$(R^6O)_mM(ACA)_n$ (VI)

in der $R^6$ eine Alkylgruppe mit 1 bis 4 Kohlenstoffatomen, ACA einen Acetylacetonatrest und m die Zahl 0 und n die Zahl 4 oder m die Zahl 2 und n die Zahl 2 bedeuten.

Gemäß einer weiteren vorteilhaften Ausführungsform der Erfindung verwendet man als Katalysatoren Polykondensationsprodukte von Estern der Titansäure mit Monoalkanolen mit 2 bis 10 Kohlenstoffatomen mit Pentaerythrit.

Gemäß einer weiteren vorteilhaften Ausführungsform der Erfindung verwendet man die erfindungsgemäß einzusetzenden Katalysatoren in einer Menge von 0,1 bis 10, vorzugsweise 0,5 bis 5, insbesondere 0,5 bis 3 Mol-%, bezogen auf vorhandene Carbonsäurereste.

Gemäß einer weiteren vorteilhaften Ausführungsform der Erfindung führt man die Kondensationsreaktion bei 150 bis 270 °C durch, wobei man die Reaktion vorzugsweise unter vermindertem Druck, insbesondere bei 5 bis 50 hPa sowie unter Schutzgas durchführt. Man kann das gebildete Reaktionswasser zusammen mit den Dihydrooxazinen abdestillieren und von diesen während der Destillation trennen, wobei man mitgeschlepptes Restwasser, insbesondere bei kürzerkettig substituierten Dihydrooxazinen, mit üblichen Trocknungsmitteln wie wasserfreiem Natriumsulfat oder Molekularsieb (4A) abtrennt. Es ist jedoch auch möglich, das Reaktionswasser durch azeotrope Destillation mittels hochsiedender Schleppmittel wie z.B. Tetralin oder Cumol aus dem Reaktionsgemisch vor der eigentlichen Destillation der Dihydrooxazine zu entfernen.

Gemäß einer weiteren bevorzugten Ausführungsform der Erfindung kann man die Dihydrooxazine direkt aus Carbonsäuren bzw. Carbonsäureestern der allgemeinen Formel VII

$R^1\text{-}COOR^7$ (VII)

in der $R^1$ wie oben definiert ist und $R^7$ Wasserstoff oder eine Alkylgruppe mit 1 bis 4 Kohlenstoffatomen bedeutet, oder aus Glyceriden, insbesondere Triglyceriden der Carbonsäuren der Formel $R^1\text{-}COOH$, herstellen, indem man diese in einer ersten Reaktionsstufe mit 3-Amino-1-propanol bei erhöhter Temperatur zu den Carbonsäure-(3-hydroxypropyl)-amiden umsetzt und das Reaktionsprodukt in einer zweiten Reaktionsstufe nach weiterer Steigerung der Reaktionstemperatur unter Ringschluß reagieren läßt. Hierbei sind als Ausgangsprodukte die technisch leicht zugänglichen Fettsäuremethylester und -glyceride bevorzugt. In einer ersten Reaktionsstufe setzt man das Ausgangsmaterial bei 100 bis weniger als 180 °C und atmosphärischem Druck in Gegenwart der vorgesehenen Katalysatoren mit 3-Amino-1-propanol zu Fettsäure-(3-hydroxypropyl)-amiden um. 3-Amino-1-propanol wird dabei in mindestens stöchiometrischen Mengen zu den vorhandenen Carbonsäureresten eingesetzt, vorzugsweise in einem Überschuß von 50 bis 400 Mol-%. Das bei der Reaktion mit Carbonsäuren entstehende Wasser und der bei der Reaktion mit Carbonsäurealkylestern entstehende Alkohol wird laufend aus dem Reaktionsgemisch abdestilliert. Überschüssiges 3-Amino-1-propanol und das bei der Reaktion mit Carbonsäureglyceriden freigesetzte Glycerin wird nach dem Ende der Reaktion unter vermindertem Druck abdestilliert. Das Reaktionsprodukt wird in einer zweiten Reaktionsstufe auf 180 bis 270 °C erhitzt, um den Ringschluß zum Dihydrooxazin zu vollziehen. Die zweite Reaktionsstufe wird bei dieser Verfahrensvariante bevorzugt unter vermindertem Druck, insbesondere bei 5 bis 50 hPa, durchgeführt. Das gebildete Reaktionswasser wird vorzugsweise zusammen mit den Dihydrooxazinen unter weiterer Auftrennung während der Destillation laufend aus dem Reaktionsgemisch abgezogen. Das Reaktionswasser kann auch vor der Destillation der Dihydrooxazine durch azeotrope Destillation entfernt werden.

Besonders vorteilhaft ist es hier, die erste und zweite Reaktionsstufe als Ein-Topf-Verfahren in einem Reaktor durchzuführen.

Die Katalysatoren werden bevorzugt in einer Menge von 0,01 bis 10, vorzugsweise 0,5 bis 5, insbesondere 0,5 bis 3 mol-%, bezogen auf im Ausgangsmaterial vorhandene Carbonsäurereste, verwendet. Dabei werden wiederum bevorzugt Titantetraalkoholate, insbesondere ausgewählt von der aus Titantetraethylat, -tetrapropylat, -tetraisopropylat und -tetrabutylat gebildeten Gruppe, eingesetzt. Alternativ werden gemischte Anhydride der Titansäure mit Monocarbonsäuren, insbesondere solcher mit 1 bis 4 Kohlenstoffatomen, vorzugsweise Titantetraacetat, verwendet. Weiterhin hier bevorzugte Katalysatoren sind Titan-bzw. Zirkoniumacetylacetonate der allgemeinen Formel IV sowie die oben erwähnten Polykondensationsprodukte von Estern der Titansäure mit Monoalkanolen mit 2 bis 10 Kohlenstoffatomen mit Pentaerythrit sowie Titan (IV)- bzw. Zr(IV)-tetra-3-amino-1-propanolate.

Weiterhin betrifft die Erfindung 5,6-Dihydro-4H-1,3-oxazine der allgemeinen Formel I, in der $R^1$ ein Kohlenwasserstoffrest aus der von 11-Hydroxy-8-heptadecenyl und 11,8- bzw. 11,9-Heptadecadienyl gebildeten Gruppe ist, als neue Verbindungen, die sich als Monomere bzw. Comonomere in Polymerisationsreaktionen einsetzen lassen.

2-(11-Hydroxy-8-heptadecenyl)-5,6-dihydro-4H-1,3-oxazin ist gemäß dem Verfahren der Erfindung aus Ricinolsäure bzw. Estern, Glyceriden oder 1-Propanol-3-amiden derselben erhältlich. Im allgemeinen wird es aus technischer Ricinusfettsäure bzw. deren Derivaten hergestellt, die kleinere Anteile an anderen Fettsäuren enthält und ungefähr die folgende Zusammensetzung aufweist:

| | |
|---|---|
| $C_{16}$ gesättigt | : 1 - 2 % |
| $C_{18}$ gesättigt | : 1 - 2 % |
| $C_{18}$ einfach ungesättigt | : Spuren - 8,5 % |
| Ricinolsäure (12-Oxyoctadecensäure) | : 86 - 92 % |
| $C_{18}$ zweifach ungesättigt | : 3 - 6 % |

2-[11,8(11,9)-Heptadecadienyl]-5,6-dihydro-4H-1,3-oxazin ist analog zu der vorstehend beschriebenen Weise aus Ricinenfettsäure erhältlich, welche ihrerseits bei der Abspaltung von 1 Mol Wasser aus Ricinolsäure bzw. technischer Ricinusfettsäure entsteht.

2-(11,8-Heptadecadienyl)-5,6-dihydro-4H-1,3-oxazin ist nach dem Verfahren der Erfindung aus Linolsäure bzw. Estern oder Ethanolamiden derselben erhältlich. Zweckmäßigerweise wird auch hier nicht eine reine Linolsäure, sondern ein aus pflanzlichen Ölen, insbesondere Sojaöl, erhältliches technisches, linolsäurereiches Fettsäuregemisch eingesetzt, das üblicherweise die folgende Zusammensetzung aufweist:

| | |
|---|---|
| $C_{10}$ gesättigt | : 0 - 0,5 % |
| $C_{12}$ gesättigt | : 0 - 0,5 % |
| $C_{14}$ gesättigt | : 0 - 2 % |
| $C_{16}$ gesättigt | : 8 - 13 % |
| $C_{18}$ gesättigt | : 3 - 6 % |
| $C_{18}$ einfach ungesättigt | : 23 - 30 % |
| $C_{18}$ zweifach ungesättigt | : 40 - 50 % |
| $C_{18}$ dreifach ungesättigt | : 4 - 12 % |
| $C_{20}$ einfach ungesättigt ) <br> ) <br> $C_{22}$ gesättigt ) | : 0 - 2 % |

Die Erfindung wird im folgenden anhand von bevorzugten Ausführungsbeispielen näher erläutert.

Beispiel 1.

2-Undecyl-5,6-dihydro-4H-1,3-oxazin

In einer Rührapparatur mit Fraktionierkolonne, absteigendem Kühler mit Destillationsvorlagen, Vakuumeinrichtung mit Kühlfalle für das Reaktionswasser, Thermometer und Gaseinleitungsrohr für Schutzgas wurde eine Mischung aus
218,7 g (1,0 Mol) Laurinsäuremethylester (98 %-ig)
151,7 g (2 Mol) 3-Amino-1-propanol (99 %-ig) und
10,3 g (0,03 Mol, 3 Mol-%) Titantetrabutylat
unter Stickstoff auf 138 bis 178 °C erhitzt. Das dabei entstehende Methanol wurde bei Normaldruck abdestilliert.

Anschließend wurde im Vakuum bei 68 bis 89 °C und 19 hPa das überschüssige 3-Amino-1-propanol abdestilliert.

Das so erhaltene Laurinsäure-(3-hydroxypropyl)-amid wurde im Vakuum auf eine Sumpftemperatur von 201 bis 238 °C erhitzt, wobei bei 180 bis 188 °C und 19 hPa das Titelprodukt und Wasser gleichzeitig überdestillierten. Das Wasser wurde zum größten Teil in der Kühlfalle kondensiert. Um das überdestillierte Dihydrooxazin von eventuell mitgerissenem Wasser zu befreien, wurde in die Destillationsvorlage als Trockenmittel Natriumsulfat gegeben.

Man erhielt 131,4 g (55 % der Theorie, bezogen auf eingesetzten Laurinsäuremethylester) der Titelverbindung, die noch geringe Mengen an Laurinsäurepropanolamid enthielt; $n_D^{20}$ = 1,4652.

Durch erneute Destillation (Kp = 99 bis 104 °C bei 0,1 hPa) wurde die reine Titelverbindung erhalten.
IR: 1675 (C=N); 1236, (C-O-C=); 1163, 1132, 1115, 1088 $cm^{-1}$ (Oxazin).
H-NMR: delta (in ppm) = 4,13 (2H,t,J=4 Hz), 3,35 (2H,t,J=4Hz), 2,12 (2H, t,J = 7 Hz), 1,85 (2H,p,J=4Hz), 1,56 (2H,br p,J=7Hz), 1,26 (16H,br s), 0,88 (3H,t,J=5Hz).

Beispiel 2.

2-(Sojaalkenyl)-5,6-dihydro-4H-1,3-oxazin.

Analog zu der Arbeitsweise des Beispiels 1 wurde folgendes umgesetzt:
219,2 g (0,75 Mol) handelsüblicher Sojafettsäuremethylester, Verseifungszahl 192
113,8 g (1,5 Mol) 3-Amino-1-propanol (99 %-ig)
7,6 g (0,023 Mol, 3 Mol-%) Titantetrabutylat.
Ausbeute = 106,3 g der Titelverbindung
Kp = 208 °C (0,04 hPa).
IR: 1675 (C=N); 1237, (C-O-C=); 1164, 1132, 1115, 1088 $cm^{-1}$ (Oxazin).
H-NMR: delta (in ppm) = 5,36 (ca. 3H,m), 4,13 (2H,t,J=4 Hz), 3,35 (2H,t,J=4Hz), 2,77 (ca. 1,5H,br,t,J = 5 Hz), 2,12 (2H, t,J = 7 Hz), 2,05 (ca. 5H,m), 1,85 (2H,p,J=4Hz), 1,56 (2H,br p,J=7Hz), 1,3 (ca. 18 H,br s), 0,88 (3H,m).

Beispiel 3.

Herstellung von 2-(11-Hydroxy-8-heptadecenyl)-5,6-dihydro-4H-1,3-oxazin aus Rizinusölsäuremethylester.

Analog der Herstellung zum Beispiel 1 wurde Rizinusölsäuremethylester mit 3-Amino-1-propanol zum 2-(11-Hydroxy-8-heptadecenyl)-5,6-dihydro-4H-1,3-oxazin umgesetzt. Die Ansatzmengen waren:
233,0 g (0,75 Mol) Rizinusölsäuremethylester; VZ = 180,5.
113,8 g (1,5 Mol) 3-Amino-1-propanol, 99 %-ig
7,6 g (0,022 Mol, 3 Mol-%) Titan-tetrabutylat.
Ausbeute = 83,3 g (33 % d. Th.) 2-(11-Hydroxy-8-heptadecenyl)-5,6-dihydro-4H-1,3-oxazin.
Kp = 210 bis 228 °C (0,05 bis 0,13 hPa)
$n_D^{20}$ = 1,4823
IR: 1672 (C=N); 1239 (C-O-C=); 1163, 1132, 1084 $cm^{-1}$ (Oxazin).
$^1$H-NMR; delta (in ppm) = 6,3-5,0 (ca. 2,5H,m); 4,13 (2H,t,J = 4 Hz); 3,60 (ca. 0,4H,br p,J = 5 Hz); 3,35 (2H,t,J = 4Hz); 2,21 (ca. 0,8H,t,J = 5 Hz); 2,10 (2H,t,J = 7 Hz); 2,1-2,0 (ca. 2H,m); 1,84 (2H,p,J = 4 Hz);

1,55 (2H,m); 1,5-1,2 (18H,br s); 0,89 (3H,t,J = 5 Hz); enthielt ca. 1/3 eliminiertes Produkt.

Beispiel 4.

Herstellung von 2-Phenyl-5,6-dihydro-4H-1,3-oxazin aus Benzoesäuremethylester.

Analog der Herstellung zum Beispiel 1 wurde Benzoesäuremethylester mit 3-Amino-1-propanol zum 2-Phenyl-5,6-dihydro-4H-1,3-oxazin umgesetzt. Die Ansatzmengen waren:
206,3 g (1,50 Mol) Benzoesäuremethylester
227,6 g (3,0 Mol) 3-Amino-1-propanol, 99 %-ig
15,3 g (0,045 Mol, 3 Mol-%) Titan-tetrabutylat
Ausbeute = 187,7 g (78 % d. Th.) 2-Phenyl-5,6-dihydro-4H-1,3-oxazin.
$n_D^{20}$ = 1,5666
Kp = 170 bis 178 °C (19 hPa)
IR: 1654 (C = N); 1273 (C-O-C =); 1134, 1106, 1072 $cm^{-1}$ (Oxazin).
$^1$H-NMR: delta (in ppm) = 7,90 (2H,dd,J = 6; 2 Hz); 7,48 (3H,m); 4,35 (2H,t,J = 5Hz); 3,60 (2H,t,J = 5Hz); 1,97 (2H,p,J = 5 Hz).

Beispiel 5.

Herstellung von 2-(3-Heptyl)-5,6-dihydro-4H-1,3-oxazin aus 2-Ethylhexansäure.

Analog der Herstellung zum Beispiel 1 wurde 2-Ethylhexansäure mit 3-Amino-1-propanol zum 2-(3-Heptyl)-5,6-dihydro-4H-1,3-oxazin umgesetzt. Die Ansatzmengen waren:
218,5 g (1,5 Mol) 2-Ethylhexansäure
227,6 g (3,0 Mol) 3-Amino-1-propanol, 99 %-ig
15,3 g (0,045 Mol, 3 Mol-%) Titan-tetrabutylat.
Ausbeute = 152 g (55 % d. Th.)
2-(3-Heptyl)-5,6-dihydro-4H-1,3-oxazin.
Kp = 138 bis 152 °C (19 hPa)

Durch erneute Destillation (Kp = 55 °C bei 1,3 hPa) wurde reines 2-(3-Heptyl)-5,6-dihydro-4H-1,3-oxazin erhalten.
Analyse:
$C_{11}H_{21}NO$ (183,296)
$C_{ber}$ = 72,08
$H_{ber}$ = 11,55
$N_{ber}$ = 7,64
$C_{gef}$ = 71,8
$H_{gef}$ = 11,6
$N_{gef}$ = 7,87
IR: 1669 (C = N); 1233 (C-O-C =); 1171, 1135, 1084 $cm^{-1}$ (Oxazin).
$^1$H-NMR: delta (in ppm) = 4,13 (2H,t,J = 5 Hz); 3,38 (2H,t,J = 5 Hz); 2,0 (1H,m); 1,85 (2H,p,J = 5 Hz); 1,5 (4H,br m); 1,3 (4H,m); 0,89 (6H,t,J = 5 Hz).

Beispiel 6.

Herstellung von 2-(cis-8-Heptadecenyl)-5,6-dihydro-4H-1,3-oxazin aus Sonnenblumenfettsäuremethylester, dessen Fettsäureanteil zu 85 % aus Ölsäure bestand.

Analog der Herstellung zum Beispiel 1 wurde Sonnenblumenfettsäuremethylester mit 3-Amino-1-propanol zum 2-(cis-8-Heptaecenyl)-5,6-dihydro-4H-1,3-oxazin umgesetzt. Die Ansatzmengen waren:
297,1 g (1,0 Mol) Sonnelblumenfettsäuremethylester SZ = 0,3; VZ = 188,8; JZ = 85,1; OHZ = 0.

151,7 g (2,0 Mol) 3-Amino-1-propanol, 99 %-ig
10,2 g (0,030 Mol, 3 Mol-%) Titan-tetrabutylat
Ausbeute = 226 g (70 % d. Th.) 2-cis-8-Heptadecenyl)-5,6-dihydro-4H-1,3-oxazin.
Kp = 205 bis 250 °C (0,04 bis 0,05 hPa)
$n_D^{20}$ = 1,4740
IR: 1675 (C=N); 1237 (C-O-C=); 1164, 1122, 1084 $cm^{-1}$ (Oxazin).
$^1$H-NMR: delta (in ppm) = 5,34 (2H,t,J = 4 Hz); 4,13 (2H,t,J = 4 Hz); 3,35 (2H,t,J = 4 Hz); 2,0 (4H,m); 1,84 (2H,p,J = 4 Hz); 1,55 (2H,p,J = 6 Hz); 1,3 (20 H,m); 0,89 (3H,t,J = 5 Hz).

Beispiel 7.

Herstellung von 2-Undecyl-5,6-dihydro-4H-1,3-oxazin aus Laurinsäuremethylester in Gegenwart von Zirkon-tetrabutylat.

Analog der Herstellung zum Beispiel 1 wurde Laurinsäuremethylester mit 3-Amino-1-propanol zum 2-Undecyl-5,6-dihydro-4H-1,3-oxazin umgesetzt. Die Ansatzmengen waren:
218,7 g (1,0 Mol) Laurinsäuremethylester
151,4 g (2,0 Mol) 3-Amino-1-propanol, 99 %-ig
11,5 g (0,03 Mol, 3 Mol-%) Zirkon-tetrabutylat
Ausbeute = 103 g (43 % d. Th.) 2-Undecyl-5,6-dihydro-4H-1,3-oxazin.
Kp = 176 bis 181 °C (17 hPa)
Das $^1$H-NMR und IR standen im Einklang mit der Struktur.

Beispiel 8.

Herstellung von 2-Sojafettalkenyl-5,6-dihydro-4H-1,3-oxazin aus Sojaöl.

In einer Rührapparatur mit Fraktionierkolonne, absteigendem Kühler mit Destillationsvorlagen, Vakuumeinrichtung mit Kühlfalle für das Reaktionswasser, Thermometer und Gaseinleitungsrohr für Schutzgas wurde eine Mischung aus
434,2 g (0,5 Mol) Sojaöl, entschleimt; VZ = 193,8
227,6 g (3,0 Mol) 3-Amino-1-propanol, 99 %-ig
15,3 g (0,045 Mol, 3 Mol-%) Titan-tetrabutylat
vorgelegt.

Nach 4 Stunden Rückfluß bei Normaldruck und unter Stickstoff wurde im Vakuum bei 81 bis 160 °C und 17 hPa das überschüssige 3-Amino-1-propanol abdestilliert. Anschließend wurde im Vakuum bei 142 bis 186 °C und 0,03 bis 0,09 hPa das freigewordene Glycerin abdestilliert.

Das so erhaltene Sojafettsäurepropanolamid wurde im Vakuum auf 266 bis 298 °C erhitzt, wobei bei 228 bis 238 °C und 0,08 bis 0,2 hPa das 2-Sojafettalkenyl-5,6-dihydro-4H-1,3-oxazin und Wasser gleichzeitig überdestillierten. Das Wasser wurde in der Kühlfalle kondensiert.

Es wurden 243 g (51 % d. Th., bezogen auf eingesetztes Sojaöl) an 2-Sojafettalkenyl-5,6-dihydro-4H-1,3-oxazin erhalten, das noch geringe Mengen an Sojafettsäurepropanolamid enthielt.

Das $^1$H-NMR und IR standen im Einklang mit der Struktur.

Beispiel 9.

Herstellung von 2-Methoxymethyl-5,6-dihydro-4H-1,3-oxazin aus Methoxyessigsäure.

Analog der Herstellung zum Beispiel 1 wurde Methoxyessigsäure mit 3-Amino-1-propanol zum 2-Methoxymethyl-5,6-dihydro-4H- 1,3-oxazin umgesetzt. Die Ansatzmengen waren:
270,3 g (3 Mol) Methoxyessigsäure
455,2 g (6,0 Mol) 3-Amino-1-propanol, 99 %-ig

30,6 g (0,09 Mol, 3 Mol-%) Titan-tetrabutylat
Ausbeute = 272,4 g (70 % d. Th.) 2-Methoxymethyl-5,6-dihydro-4H-1,3-oxazin.
Kp = 110 bis 127 °C (127 bis 133 hPa)
IR: 1673 (C=N); 1249 (C-O-C=); 1129, 1095, 1067 $cm^{-1}$ (Oxazin).
$^{1}$H-NMR: delta (in ppm) = 4,22 (2H,t,J = 5 Hz); 3,87 (2H,s); 3,43 (2H,t,J = 5 Hz); 3,41 (3H,s); 1,91 (2H,p,J = 5Hz).

**Ansprüche**

1. Verfahren zur Herstellung von 5,6-Dihydro-4H-1,3-oxazinen der allgemeinen Formel I

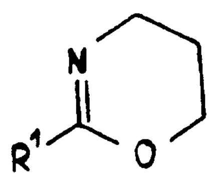

(I)

in der $R^1$
ein gegebenenfalls hydroxy, vicinal dihydroxy- oder vicinal hydroxy, $C_1$-$C_2$-alkoxysubstituierter Kohlenwasserstoffrest mit mindestens 4, insbesondere 7 bis 21 Kohlenstoffatomen in der Kohlenwasserstoffkette,
ein gegebenenfalls im aromatischen Kern substituierter Aryl- oder Aralkylrest,
eine hydroxysubstituierte Alkylgruppe mit 1 bis 3 Kohlenstoffatomen,
eine alkoxysubstituierte Alkylgruppe mit 1 bis 6 Kohlenstoffatomen in der Alkyl- und 1 bis 18 Kohlenstoffatomen in der Alkoxygruppe, oder
einen Rest der allgemeinen Formel II
$R^2$-$(OC_2H_5)_p$-O-$CH_2$-     (II)
in der
$R^2$ eine Alkyl- oder Alkenylgruppe mit 1 bis 18 Kohlenstoffatomen, insbesondere eine Methylgruppe, und
p eine Zahl im Bereich von 1 bis 10, insbesondere 1 bis 2, ist,
bedeutet,
durch Kondensation von Carbonsäure-(3-hydroxypropyl)-amiden bzw. deren Vorläufern in Gegenwart von Katalysatoren in flüssiger Phase, dadurch gekennzeichnet, daß man
a) Carbonsäuren der allgemeinen Formel III
$R^1$-COOH     (III)
in der $R^1$ wie oben definiert ist, Ester dieser Carbonsäuren mit Monoalkanolen mit 1 bis 4 Kohlenstoffatomen oder Glyceride dieser Carbonsäuren mit 3-Amino-1-propanol oder
b) 3-Hydroxypropyl-amide dieser Carbonsäuren
in Gegenwart von Titan- bzw. Zirkoniumverbindungen der allgemeinen Formel IV
$M(OR^3)_4$     (IV)
in der M vierwertiges Titan oder Zirkonium und $R^3$ eine Alkylgruppe mit mindestens 2, insbesondere 2 bis 4 Kohlenstoffatomen, eine Acylgruppe mit mindestens 2, insbesondere 2 bis 10 Kohlenstoffatomen oder eine 3-Aminopropylen-1-oxygruppe oder einen Rest eines beta-Diketons der allgemeinen Formel V bedeuten,
$R^4$-C =CH-CO-$R^5$     (V)
in der $R^4$ und $R^5$ gleiche oder verschiedene Reste aus der von Alkylgruppen mit 1 bis 4 Kohlenstoffatomen und gegebenenfalls in p-Stellung substituiertem Phenyl gebildeten Gruppe sind, wobei jeweils zwei der Gruppen $R^3$ zusammen aus dem zweibindigen Rest eines zweiwertigen Alkohols mit 2 bis 4 Kohlenstoffatomen bestehen können,
in Gegenwart von Titanylacetylacetonat oder
in Gegenwart von Kondensationsprodukten von Titan(IV) bzw. Zirkonium(IV)-tetraalkoxylaten der allgemeinen Formel III, in der M und $R^3$ wie oben definiert sind, mit mehrfunktionellen Alkanolen, insbesondere mit 3 bis 12 Kohlenstoffatomen und 2 bis 6 Hydroxylgruppen,
kondensiert und die so erhaltenen 5,6-Dihydro-4H-1,3-oxazine unter Entfernung des Reaktionswassers bzw. -alkohols isoliert.

2. Verfahren nach Anspruch 1, dadurch gekennzeichnet, daß man als Katalysatoren Ester der Titansäure ($H_4TiO_4$) oder Zirkonsäure ($H_4ZrO_4$) bzw. gemischte Anhydride der Titansäure oder Zirkonsäure mit organischen Säuren der allgemeinen Formel III, in der M = Ti oder Zr und $R^3$ eine Alkylgruppe mit 2 oder

mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten, verwendet.

3. Verfahren nach Anspruch 1 oder 2, dadurch gekennzeichnet, daß man Katalysatoren aus der von Titan- bzw. Zirkonium tetraethylat, -tetrapropylat, -tetraisopropylat, -tetrabutylat und -tetraacetat gebildeten Gruppe verwendet.

4. Verfahren nach Anspruch 1 oder 2, dadurch gekennzeichnet, daß man Tetra(3-amino-1-propyl)-titanat oder -zirkonat als Katalysator verwendet.

5. Verfahren nach Anspruch 1 oder 2 , dadurch gekennzeichnet, daß man als Katalysatoren Titan- bzw. Zirkonium-acetylacetonate der allgemeinen Formel VI

$(R^6O)_mM(ACA)_n$ (VI)

in der $R^6$ eine Alkylgruppe mit 1 bis 4 Kohlenstoffatomen, ACA einen Acetylacetonatrest und m die Zahl 0 und n die Zahl 4 oder m die Zahl 2 und n die Zahl 2 bedeuten, verwendet.

6. Verfahren nach Anspruch 1 oder 2, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

7. Verfahren nach mindestens einem der Ansprüche 1 bis 6, dadurch gekennzeichnet, daß man die Katalysatoren in Mengen von 0,1 bis 10, vorzugsweise 0,5 bis 5, insbesondere 0,5 bis 3 Mol, bezogen auf vorhandene Carbonsäurereste, verwendet.

8. Verfahren nach mindestens einem der Ansprüche 1 bis 7, dadurch gekennzeichnet, daß man die Kondensation bei 150 bis 270 °C durchführt.

9. Verfahren nach mindestens einem der Ansprüche 1 bis 8, dadurch gekennzeichnet, daß man die Reaktion unter vermindertem Druck durchführt.

10. Verfahren nach mindestens einem der Ansprüche 1 bis 9, dadurch gekennzeichnet, daß man das bei der Reaktion gebildete Wasser bzw. Alkanol oder Glycerin zusammen mit den Dihydrooxazinen abdestilliert und anschließend oder gleichzeitig von diesen trennt oder vor der Destillation der Dihydrooxazine durch gegebenenfalls azeotrope Destillation entfernt.

11. Verfahren nach mindestens einem der Ansprüche 1 bis 10, dadurch gekennzeichnet, daß man in einer ersten Reaktionsstufe Carbonsäuren bzw. Carbonsäureester der allgemeinen Formel VII

$R^1$-$COOR^7$ (VII)

in der $R^1$ wie oben definiert ist und $R^7$ Wasserstoff oder eine Alkylgruppe mit 1 bis 4 Kohlenstoffatomen bedeutet, oder Carbonsäureglyceride, insbesondere Triglyceride, in Gegenwart von 3-Amino-1-propanol bei erhöhter Temperatur zu den Carbonsäure-(3-hydroxypropyl)-amiden umsetzt und das Reaktionsprodukt in einer zweiten Reaktionsstufe nach weiterer Steigerung der Reaktionstemperatur unter Ringschluß reagieren läßt.

12. Verfahren nach Anspruch 11, dadurch gekennzeichnet, daß man das 3-Amino-1-propanol in einem 50 bis 400 Mol-%-igem Überschuß, bezogen auf vorhandene Carbonsäurereste, verwendet.

13. Verfahren nach Anspruch 11 oder 12, dadurch gekennzeichnet, daß man vor der zweiten Reaktionsstufe nicht umgesetztes bzw. überschüssiges 3-Amino-1-propanol sowie gebildetes Wasser und gegebenenfalls Alkohol oder Glycerin aus dem Reaktionsgemisch entfernt.

14. Verfahren nach mindestens einem der Ansprüche 11 bis 13, dadurch gekennzeichnet, daß man in der ersten Reaktionsstufe eine Reaktionstemperatur von 100 bis weniger als 180 °C verwendet.

15. Verfahren nach mindestens einem der Ansprüche 11 bis 14, dadurch gekennzeichnet, daß man in der zweiten Reaktionsstufe eine Reaktionstemperatur von 180 bis 270 °C verwendet.

16. Verfahren nach mindestens einem der Ansprüche 11 bis 15, dadurch gekennzeichnet, daß man als Titansäureester Titantetraalkoholate verwendet.

17. Verfahren nach mindestens einem der Ansprüche 11 bis 16, dadurch gekennzeichnet, daß man Titantetraalkoholate aus der von Titantetraethylat, -tetrapropylat, -tetraisopropylat und -tetrabutylat gebildeten Gruppe verwendet.

18. Verfahren nach mindestens einem der Ansprüche 11 bis 15, dadurch gekennzeichnet, daß man Tetra(3-amino-1-propyl)-titanat bzw. -zirkonat als Katalysator verwendet.

19. Verfahren nach mindestens einem der Ansprüche 11 bis 15, dadurch gekennzeichnet, daß man gemischte Anhydride der Titansäure mit Monocarbonsäuren verwendet.

20. Verfahren nach mindestens einem der Ansprüche 11 bis 15, dadurch gekennzeichnet, daß man Titantetraacetat verwendet.

21. Verfahren nach mindestens einem der Ansprüche 11 bis 15, dadurch gekennzeichnet, daß man als Katalysatoren Titan-bzw. Zirkoniumacetylacetonate der allgemeinen Formel VI

$(R^6O)_mM(ACA)_n$ (VI)

verwendet, in der $R^6$, ACA, m und n wie oben definiert sind.

22. Verfahren nach mindestens einem der Ansprüche 11 bis 15, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

23. Verfahren nach einem der Ansprüche 11 bis 22, dadurch gekennzeichnet, daß man die Katalysatoren in einer Menge von 0,01 bis 10, vorzugsweise 0,5 bis 5, insbesondere 0,5 bis 3 Mol-%, bezogen auf vorhandene Carbonsäurereste, zusetzt.

24. Verfahren nach mindestens einem der Ansprüche 11 bis 23, dadurch gekennzeichnet, daß man die Reaktion unter vermindertem Druck durchführt.

25. Verfahren nach mindestens einem der Ansprüche 11 bis 24, dadurch gekennzeichnet, daß man das in der zweiten Reaktionsstufe gebildete Wasser zusammen mit den 5,6-Dihydro-4H-1,3-oxazinen abdestilliert und anschließend oder gleichzeitig entfernt oder vor der Destillation der Dihydrooxazine durch gegebenenfalls azeotrope Destillation entfernt.

26. Verfahren nach mindestens einem der Ansprüche 11 bis 25, dadurch gekennzeichnet, daß man die erste und zweite Reaktionsstufe als Ein-Topf-Verfahren durchführt.

27. 5,6-Dihydro-4H-1,3-oxazine der allgemeinen Formel I,

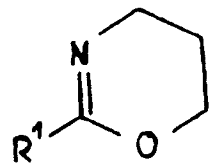

(I)

in der $R^1$ eine 11-Hydroxy-8-heptadecenyl- oder 11,8- bzw. 11,9-Heptadecadienylgruppe ist.

Europäisches Patentamt

# EUROPÄISCHER RECHERCHENBERICHT

Nummer der Anmeldung

EP 90 10 7481

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 681 333 (M.H. LITT et al.)<br>* Anspruch 1; Spalte 3, Zeilen 8-44; Spalten 5,7, Beispiel 1, Ansatz Nr. 40; Spalten 9,10, Beispiel 6; Spalte 11, Beispiel 10 * | 1 | C 07 D 265/08 |
| A | * Anspruch 10 *<br>--- | 27 | |
| D,Y | EP-A-0 219 284 (RESEARCH DEVELOPMENT CORP. OF JAPAN)<br>* ganzes Dokument *<br>--- | 1 | |
| A | DE-A-2 127 776 (CHEMISCHE WERKE HUELS AG)<br>* Ansprüche 1,2 *<br>--- | 1 | |
| A | DE-A-2 531 276 (CHEMISCHE WERKE HUELS AG)<br>* Ansprüche 2,5 *<br>--- | 1 | |
| A,D | US-A-3 470 267 (M.H. LITT)<br>* Spalte 6, Zeilen 14,15 *<br>----- | | |

RECHERCHIERTE SACHGEBIETE (Int. Cl.5)

C 07 C 265/00

Der vorliegende Recherchenbericht wurde für alle Patentansprüche erstellt

| Recherchenort | Abschlußdatum der Recherche | Prüfer |
|---|---|---|
| BERLIN | 17-07-1990 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)